# NEW EUROPEAN PATENT SPECIFICATION

(11) **EP 0 389 034 B2**
(45) Date of publication and mention of the opposition decision: **14.07.1999**
(45) Mention of the grant of the patent: 14.09.1994
(21) Application number: 90200573.5
(22) Date of filing: 12.03.1990
(51) Int. Cl.: C12N 15/40, A61K 39/187, C12Q 1/70

(54) **Hog cholera virus vaccine and diagnostic**
Impfstoff und Diagnostikum für den Schweine-Cholera-Virus
Vaccin et test de diagnostic pour le virus du choléra porcin

(30) Priority: 19.03.1989 EP 89104921
(43) Date of publication of application: 26.09.1990
(60) Divisional application: 94200200.7
(73) Proprietor: Akzo Nobel N.V., 6824 BM Arnhem (NL)
(72) Inventor: Meyers, Gregor, D-7000 Stuttgart 80 (DE); Rümenapf, Tillmann, D-7400 Tübingen (DE); Thiel, Heinz-Jürgen, D-7400 Tübingen (DE)
(74) Representative: Hermans, Franciscus G.M.

(56) References cited:
- EP-A- 0 236 977
- VIRUS RESEARCH, vol. 11, no. 4, 1988, pages 281-291, Elsevier Science Publishers B.V., Amsterdam, NL; R.J.M MOORMANN et al.: "Hog cholera virus: identification and characterization of the viral RNA and the virus-specific RNA synthesized in infected swine Kidney cells"
- THE JOURNAL OF GENERAL VIROLOGY, vol. 70, no. 2, February 1989, pages 253-266, SGM, GB; M.S. COLLETT et al.: "Recent advances in pestivirus research"
- VIROLOGY, vol. 171, no. 2, 1989, pages 555-567, Academic Press, Inc.; G. MEYERS et al.: "Molecular cloning and nucleotide sequence of the genome of hog cholera virus"
- U.Goodenough:"Genetics (2nd edition) 1978 BALTIMORE,US,
- Methods in Enzymology Volume 185 (O.V.Goeddel Ed.) SAN DIEGO US 1991
- L.A.King and R.O.Possee:The Baculovirus Expression System Cambridge G.B. 1992

## Description

The present invention is concerned with a nucleic acid sequence, a recombinant nucleic acid molecule comprising such a nucleic acid sequence, a recombinant expression system comprising such a recombinant nucleic acid molecule, a polypeptide characteristic of the hog cholera virus, a vaccine comprising such a polypeptide or recombinant expression system as well as a method for the preparation of such vaccines.

Classical swine fever or hog cholera (HC) represents an economically important disease of swine in many countries worldwide. Under natural conditions, the pig is the only animal known to be susceptible to HC. Hog cholera is a highly contagious disease which causes degeneration in the walls of capillaries, resulting in hemorrhages and necrosis of the internal organs. In the first instance hog cholera is characterized by fever, anorexia, vomiting and diarrhea which can be followed by a chronic course of the disease characterized by infertility, abortion and weak offsprings of sows. However, nearly all pigs die within 2 weeks after the first symptoms appear.

The causative agent, the hog cholera virus (HCV) has been shown to be structurally and serologically related to bovine viral diarrhea virus (BVDV) of cattle and to border disease virus (BDV) of sheep. These viruses are grouped together into the genus pestivirus within the family togaviridae. The nature of the genetic material of pestiviruses has long been known to be RNA. i.e. positive-strand RNA which lacks significant polyadenylation. The HCV probably comprises 3-5 structural proteins of which two are possibly glycosylated. The number of non-structural viral proteins is unknown.

Modified HCV vaccines (comprising attenuated or killed viruses) for combating HC infection have been developed and are presently used. However, infection of tissue culture cells to obtain HCV material to be used in said modified virus vaccines, leads to low virus yields and the virions are hard to purify. Modified live virus vaccines always involve the risk of inoculating animals with partially attenuated pathogenic HCV which is still pathogenic and can cause disease in the inoculated animal or offspring and of contamination by other viruses in the vaccine. In addition the attenuated virus may revert to a virulent state.

There are also several disadvantages using inactivated vaccines, e.g. the risk of only partial inactivation of viruses, the problem that only a low level of immunity is achieved requiring additional immunizations and the problem that antigenic determinants are altered by the inactivation treatment leaving the inactivated virus less immunogenic.

Furthermore, the usage of modified HCV vaccines is not suited for eradication programmes.

Until now, according to our knowledge diagnostic tests in swine which can distinguish between HCV or BVDV infection are not available. This is important as BVDV infection in pigs is of lower significance than HCV infection which means that BVDV infected pigs do not have to be eradicated.

Vaccines containing only the necessary and relevant HCV immunogenic material which is capable of eliciting an immune response against the pathogen do not display abovementioned disadvantages of modified vaccines.

According to the present invention a nucleic acid sequence which is a fragment of the hog cholera virus genome, and encoding a polypeptide characteristic of hog cholera virus which polypeptide comprises the amino acid sequence located within amino acid position 689-1067 has been found. Both the nucleic acid sequence and the polypeptide can be used for the preparation of a vaccine containing only the necessary and relevant immunogenic material for immunizing animals against HCV infection. "Nucleic acid sequence" refers both to a ribonucleic acid sequence and a deoxy-ribonucleic acid sequence.

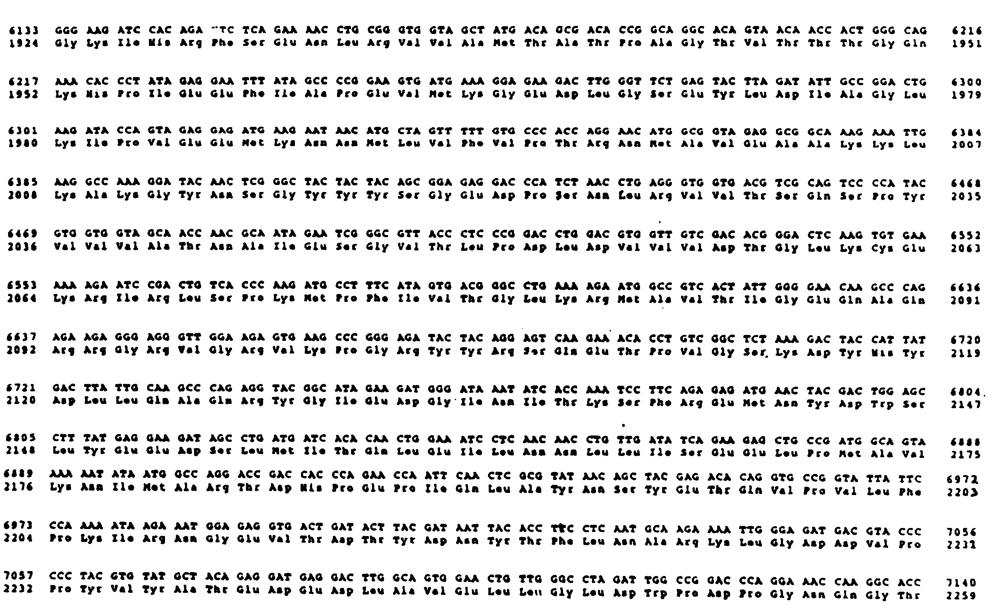

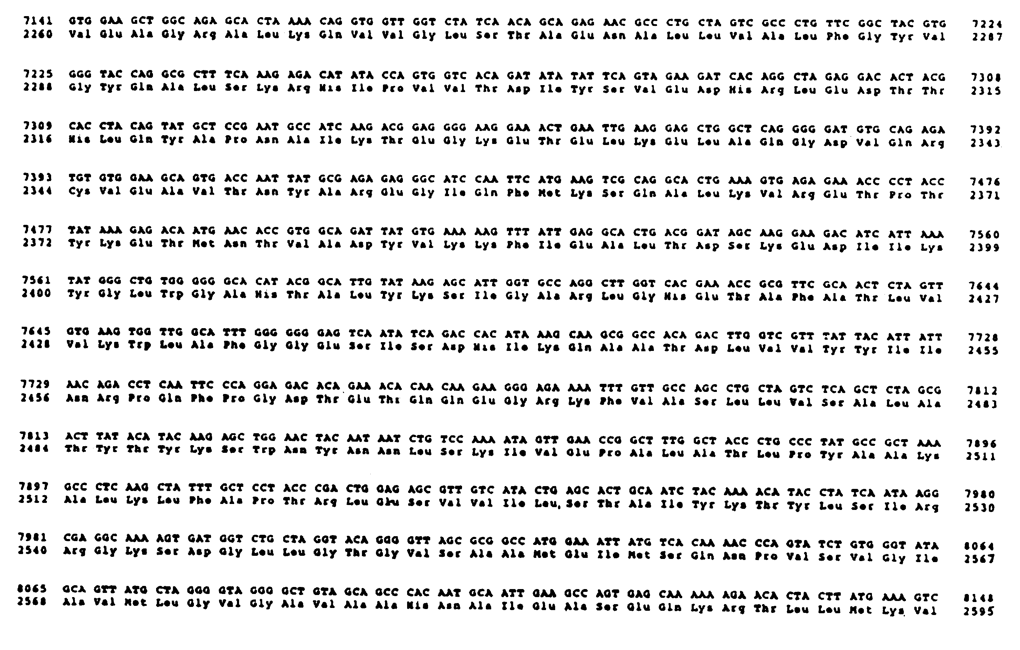

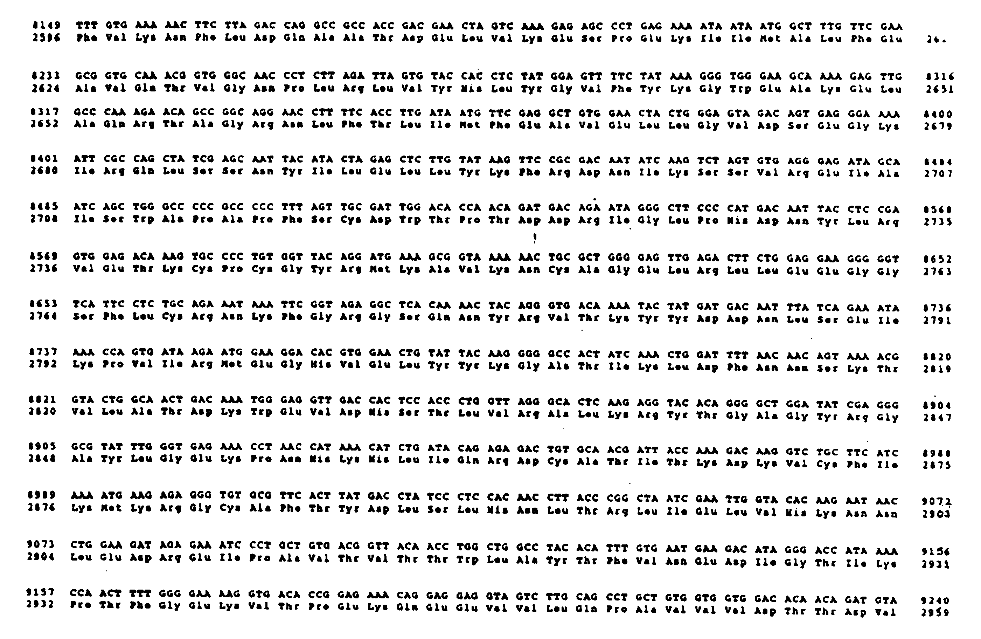

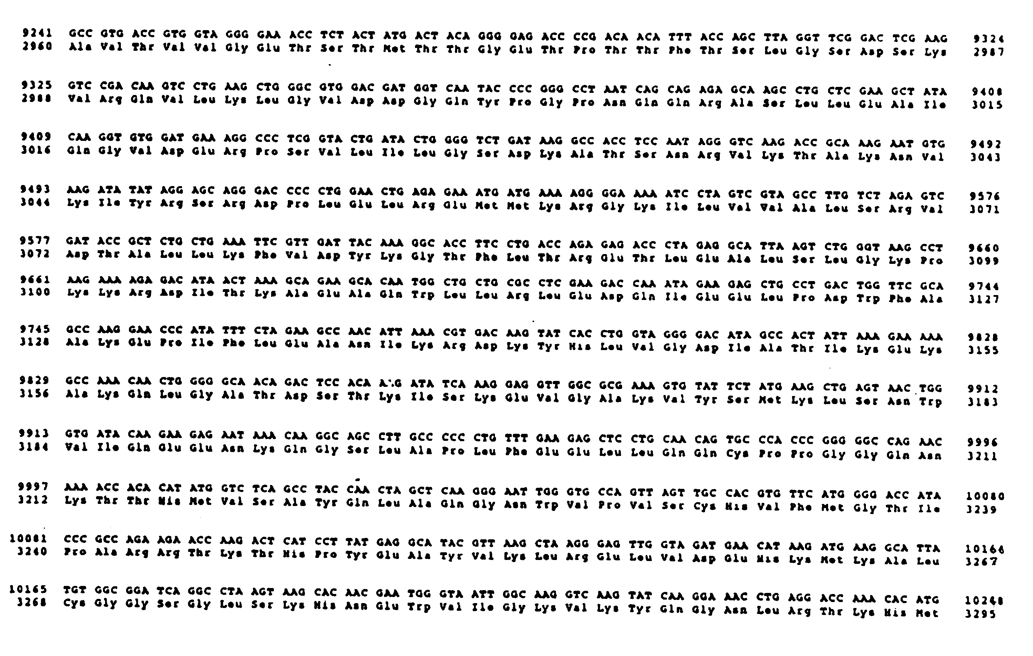

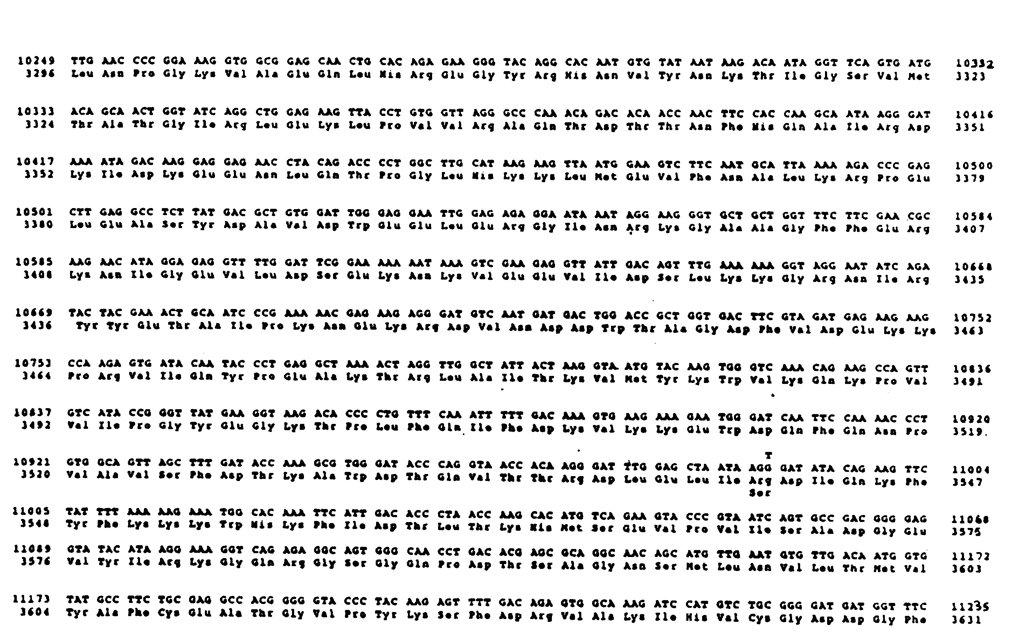

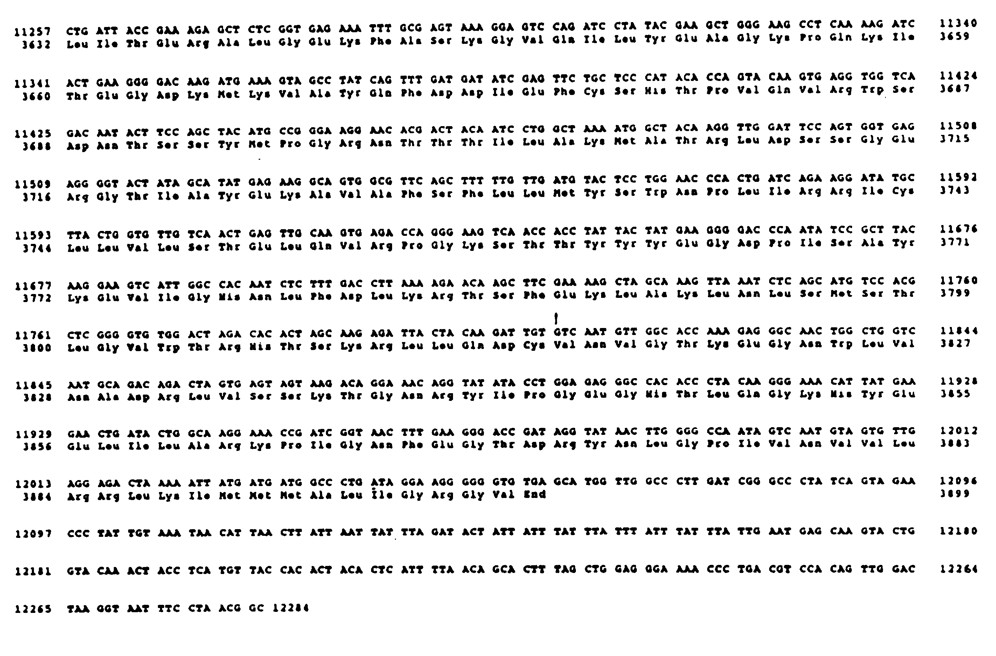

A nucleic acid sequence according to the present invention is shown in figure 2. As is well known in the art, the degeneracy of the genetic code permits substitution of bases in a codon resulting in an other codon but still coding for the same amino acid, e.g. the codon for the amino acid glutamic acid is both GAT and GAA. Consequently, it is clear that for the expression of a polypeptide with the amino acid sequence shown in figure 2 encoded by a nucleic acid sequence according to the invention, use can be made of a nucleic acid sequence with such an alternative codon composition different from the nucleic acid sequence shown in figure 2.

Also included within the scope of the invention are nucleic acid sequences which hybridize under stringent conditions to the nucleic acid sequence shown in figure 2 encoding the polypeptide comprising the amino acid sequence located within amino acid position 689-1067. These nucleic acid sequences are related to the nucleic acid sequence shown in figure 2 but may comprise nucleotide substitutions, mutations, insertions, deletions etc. and encode polypeptides which are functionally equivalent to the polypeptide defined above shown in figure 2, i.e. the amino acid sequence of a related polypeptide is not identical with the amino acid sequence shown in figure 2 but features corresponding immunological properties characteristic for HCV.

The nucleic acid sequence shown in figure 2 is a cDNA sequence derived from the genomic RNA of HCV. This continuous sequence is 12284 nucleotides in length, and contains one long open reading frame (ORF), starting with the ATG codon at position 364 to 366 and ending with a TGA codon as a translational stop codon at position 12058 to 12060. This ORF consists of 3898 codons capable of encoding 435 kDa of protein.

In vivo, during HCV replication in an infected cell, this protein is synthesized as a polyprotein precursor molecule which is subsequently processed to fragment polypeptides by (enzymatic) cleavage of the precursor molecule. These fragments form after possible post-translational modifications the structural and non-structural proteins of the virus. The nucleic acid sequence according to the present invention contains the genetic information for such a fragment with immunizing properties against HCV or immunological properties characteristic for HCV or contains the genetic information for a portion of such a fragment which still has the immunizing properties or the immunological properties characteristic for HCV.

Fragment polypeptides of the polypeptide according to figure 2 and the portions thereof, which can be used for the immunisation of animals against HC or for diagnosis of HC are located within the amino acid position about 1-249, 263-487, 488-688 or 689-1067.

The 1-249 region essentially represents the core protein whereas the 263-487, 488-688 and 689-1067 regions essentially represent glycoproteins of 33 kD, 44/48 kD and 55 kD respectively. Within the scope of the invention is a nucleic acid sequences comprising the genetic information for at least the 55 kD coding region mentioned above or portions thereof.

A preferred region to be incorporated into a vaccine against HCV infection is the region corresponding to the 55 kD protein of HCV or a portion thereof still having immunizing activity.

Furthermore, a nucleic acid sequence at least comprising the coding sequences for said 55 kD protein or portion thereof can advantageously be applied according to the present invention.

In addition, a preferred portion of the HCV 55 kD protein, which can be used for immunization of pigs against HCV infection, is determined by analyses of HCV deletion mutants with anti-55 kD protein monoclonal antibodies having virus neutralizing activity. Such a portion comprising an epitope spans the amino acid sequence about 812-859 and is coded by the nucleotide sequence about 2799-2938. A nucleic acid sequence at least comprising said nucleotide sequence forms part of the present invention too.

A nucleic acid sequence according to the invention which can be used for the diagnosis of HCV infection in pigs and which can be applied to discriminate HCV from BVDV can be derived from the gene encoding the 55 kD protein.

Preferably, such a nucleic acid sequence is derived from the nucleotide sequences 2587-2619 or 2842-2880, both sequences being part of the gene encoding the 55 kD protein. A preferred oligonucleotide for diagnostic purposes is:

5' - CCT ACT AAC CAC GTT AAG TGC TGT GAC TTT AAA - 3'
or
5' - TTC TGT TCT CAA GGT TGT GGG GCT CAC TGC TGT GCA CTC
- 3'

Moreover, a nucleic acid sequence comprising at least a sub-sequence of said oligonucleotides and which still can be used to differentiate between HCV and BVDV forms part of the invention.

The invention also relates to a test kit to be used in an assay, this test kit containing a nucleic acid sequence according to the invention.

Preferably the test kit comprises an oligonucleotide mentioned above or a nucleic acid sequence comprising at least a sub-sequence thereof.

Variations or modifications in the polypeptide shown in figure 2 or fragments thereof, such as natural variations between different strains or other derivatives, are possible while retaining the same immunologic properties. These variations may be demonstrated by (an) amino acid difference(s) in the overall sequence or by deletions, substitutions, insertions, inversions or additions of (an) amino acid(s) in said polypeptide.

Moreover, the potential exists, in the use of recombinant DNA technology, for the preparation of various derivatives of the polypeptide shown in figure 2 or fragments thereof, variously modified by resultant single or multiple amino acid substitutions, deletions, additions or replacements, for example by means of site directed mutagenesis of the underlying DNA. All nucleic acid sequences encoding such modifications resulting in derivatives of the polypeptide defined above shown in figure 2 or fragments thereof are included within the scope of the present invention so long as the essential characteristic activity of said polypeptide or fragment thereof, remains unaffected in essence.

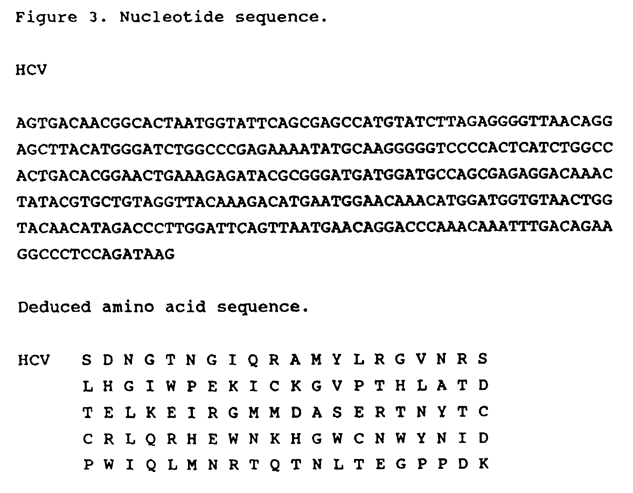

RNA isolated from pelleted virions was isolated and used for the synthesis of cDNA. This cDNA was cloned in phage kgtll and the respective library was amplified and screened with goat anti-HCV antiserum. Two positive clones could be identified and shown to have inserts with sizes of 0,8 kb and 1,8 kb. The 0,8 kb kgtll insert was partially sequenced (see figure 3) and determined to be located between about 1,2 and 2,0 kb on the HCV genome (see figure 2).

The invention also relates to a test kit to be used in an assay, this test kit containing a nucleic acid sequence according to the invention.

A nucleic acid sequence according to the present invention can be ligated to various vector nucleic acid molecules such as plasmid DNA, bacteriophage DNA or viral DNA to form a recombinant nucleic acid molecule. The vector nucleic acid molecules preferably contain DNA sequences to initiate, control and terminate transcription and translation. A recombinant expression system comprising a host containing such a recombinant nucleic acid molecule can be used to allow for a nucleic acid sequence according to the present invention to express a polypeptide encoded by said nucleic acid sequence. The host of above-mentioned recombinant expression system can be of procaryotic origin, e.g. bacteria such as E.coli, B.subtilis and Pseudomonas, viruses such as vaccinia and fowl pox virus or eucaryotic origin such as yeasts or higher eucaryotic cells such as insect, plant or animal cells.

Immunization of animals against HC can, for example, be achieved by administering to the animal a polypeptide encoded by a nucleic acid sequence according to the invention as a so-called "sub-unit" vaccine. The subunit vaccine according to the invention comprises a polypeptide generally in a pure form, optionally in the presence of a pharmaceutically acceptable carrier.

Small fragments are preferably conjugated to carrier molecules in order to raise their immunogenicity. Suitable carriers for this purpose are macromolecules, such as natural polymers (proteins, like key hole limpet hemocyanin, albumin, toxins), synthetic polymers like polyamino acids (polylysine, polyalanine), or micelles of amphiphilic compounds like saponins. Alternatively these fragments may be provided as polymers thereof, preferably linear polymers. Polypeptides to be used in such subunit vaccines can be prepared by methods known in the art, e.g. by isolation said polypeptides from hog cholera virus, by recombinant DNA techniques or by chemical synthesis.

If required the polypeptides encoded by a nucleic acid sequence according to the invention to be used in a vaccine can be modified in vitro or in vivo, for example by glycosylation, amidation, carboxylation or phosphorylation.

An alternative to subunit vaccines are "vector" vaccines. A nucleic acid sequence according to the invention is integrated by recombinant techniques into the genetic material of another micro-organism (e.g. virus or bacterium) thereby enabling the micro-organism to express a polypeptide according to the invention. This recombinant expression system is administered to the animal to be immunized whereafter it replicates in the inoculated animal and expresses the polypeptide resulting in the stimulation of the immune system of the animal. Suitable examples of vaccine vectors are pox viruses (such as vaccinia, cow pox, rabbit pox), avian pox viruses (such as fowl pox virus) pseudorabies virus, adeno viruses, influenza viruses, bacteriophages or bacteria (such as Escherichia coli and Salmonella).

The recombinant expression system having a nucleic acid sequence according to the invention inserted in its nucleic acid sequence can for example be grown in a cell culture and can if desired be harvested from the infected cells and formed to a vaccine optionally in a lyophilized form. Said genetically manipulated micro-organism can also be harvested from live animals infected with said micro-organism. Abovementioned recombinant expression system can also be propagated in a cell culture expressing a polypeptide encoded by a nucleic acid sequence according to the invention, whereafter the polypeptide is isolated from the culture.

A vaccine comprising a polypeptide encoded by a nucleic acid sequence according to the invention or a recombinant expression system according to the present invention can be prepared by procedures well-known in the art for such vaccines. A vaccine according to the invention can consist inter alia of whole host, host extract, partially or completely purified polypeptide or a partially or completely purified recombinant expression system as above-mentioned.

The vaccine according to the invention can be administered in a conventional active immunization scheme: single or repeated administration in a manner compatible with the dosage formulation and in such amount as will be therapeutically effective and immunogenic. The administration of the vaccine can be done, e.g. intradermally, subcutaneously, intramusculary, intra-venously or intranasally. For parenteral administration the vaccines may additionally contain a suitable carrier, e.g. water, saline or buffer solution with or without adjuvants, stabilizers, solubilizers, emulsifiers etc.

The vaccine may additionally contain immunogens related to other diseases or nucleic acid sequences encoding these immunogens like antigens of parvovirus, pseudorabies virus, swine influenza virus, TGE virus, rotavirus, Escherichia coli, Bordetella, Pasteurella, Erysipelas etc. to produce a multivalent vaccine.

Polypeptides encoded by a nucleic acid sequence according to the present invention can also be used in diagnostic methods to detect the presence of HCV antigen or antibody in an animal. Moreover, nucleic acid sequences according to the invention can be used to produce polypeptides to be used in above-mentioned diagnostic methods or as a hybridisation probe for the detection of the presence of HCV nucleic acid in a sample.

### Example 1

### Immunological identification of cDNA clones

Infection of cells and harvesting of virus. PK15 and 38A₁D cells were grown in DMEM with 10% FCS and were infected in suspension by the virulent HCV strain Alfort in a volume of 20-30 ml at a cell concentration of 5 x 10⁷/ml at 37 °C for 90 min with an m.o.i. of 0.01 to 0.001 (as determined by immunofluorescence assay). Thereafter, the PK15 cells were seeded in tissue culture plates (150 mm diameter), while the suspension cells 38A₁D were incubated in bottles with gentle stirring (Tecnomara, Switzerland). For cDNA synthese, the tissue culture supernatant was harvested 48 hours after infection, clarified at 12,000 g, and afterwards the virus pelleted in a TFA 20 rotor (Contron, Italy) at 54,000 g for 12 hours.

### Example 1

### Immunological identification of cDNA clones

Infection of cells and harvesting of virus. PK15 and 38A₁ D cells were grown in DMEM with 10% FCS and were infected in suspension by the virulent HCV strain Alfort in a volume of 20-30 ml at a cell concentration of 5 x 10⁷/ml at 37 °C for 90 min with an m.o.i. of 0.01 to 0.001 (as determined by immunofluorescence assay). Thereafter, the PK15 cells were seeded in tissue culture plates (150 mm diameter), while the suspension cells 38A₁D were incubated in bottles with gentle stirring (Tecnomara, Switzerland). For cDNA synthese, the tissue culture supernatant was harvested 48 hours after infection, clarified at 12,000 g, and afterwards the virus pelleted in a TFA 20 rotor (Contron, Italy) at 54,000 g for 12 hours.

Preparation of goat anti-HCV serum. A fibroblastic cell strain was established from the skin biopsy of a young goat by standard cell culture techniques. The cells were initially grown in F-10 medium with 10% FCS and later in DMEM with 10% FCS. Goat fibroblasts were infected with HCV. Over the first 26 hours p.i., the cells were washed every 8 hours 3 times with PBS and afterwards incubated in DMEM with 10% preimmune goat serum (PGS). 48 hours p.i., the tissue culture supernatant was harvested and used as stock virus. Before immunization, goat cells for 30 tissue culture dishes (150 mm diameter) were kept for 3 passages in medium with 10% PGS and then infected with the stock virus. 48 hours p.i., the goat was immunized with X-ray-inactivated pelleted virus and infected cells. Both were emulsified in Freund's adjuvant (complete for basis immunization, incomplete for booster injections) and injected subcutaneously. To obtain antibodies recognizing denatured molecules, the antigen preparations were incubated in 0.2% SDS, 3 mM DTT at 95 °C for 5 min before injection.

RNA preparation, cDNA synthesis and cloning. RNA from virions was isolated by using the guanidine thiocyanate method described by Chirgwin et al. (1979). RNA from pelleted virions (5 µg total RNA, approximately 0.5 µg HCV RNA) and 0.1 µg of random hexanucleotide primer (Pharmacia, Sweden) in 20 µl of water were heated to 65 °C for 10 min, chilled on ice, and adjusted to first strand buffer (50 mM Tris-HCl pH 8.3; 30 mM KCI; 8 mM MgCl₂; 1 mM DTT, dATP, dCTP, dGTP, dTTP 1 mM each and 500 units RNAguard [Pharmacia, Sweden] per ml) in a final volume of 32 µl. 35 units of AMV reverse transcriptase (Life Sciences Inc., USA) were added. After 1 hour at 43 °C the reaction mixture was added to one vial of second strand synthesis mixture (cDNA synthesis kit, Pharmacia, Sweden). Second strand synthesis, preparation of blunt ends, and Eco RI adaptor ligation and phosphorylation were done as recommended by the supplier.

The cDNA was size-fractionated by preparative agarose gel electrophoresis. The part of the gel containing DNA molecules smaller than 0.5 kb was discarded. The remaining DNA was concentrated by running the gel reversely for 15 min and extracted from the agarose after 3 cycles of freezing and thawing with phenol.

Ethanol co-precipitated cDNA and λgt11 DNA (1 µg EcoRI digested dephosphorylated arms, Promega, USA) was ligated by 3 units of T4 DNA ligase (Pharmacia, Sweden) in a total volume of 10 ul ligase buffer (30 mM Tris-HCI pH 7.4; 10 mM MgCl₂; 10 mM DTT; 1 mM ATP). In vitro packaging with a commercially available extract (Packagene, Promega, USA) and infection of E.coli K12 cells, strain Y 1090, with resulting phages was performed as recommended by the supplier. The library was amplified once as described (Davis et al., 1986).

Screening of λgt11 library. Screening was basically performed as described (Young and Davis, 1983) using the Protoblot system purchased from Promega, USA (Huynh et al., 1985) and a serum dilution of 10⁻³. For background reduction the goat anti HCV serum was treated with E.coli lysate (strain Y1090) at 0.8 mg/ml (Huynh et al., 1985). Two positive clones having inserts of 0.8 kb and 1.8 kb, respectively could be identified.

Nick translation and Northern hybridization. 50 ng of the 0.8 kb HCV nucleic acid sequence labeled with [α³²P]dCTP (3000 Ci per mMole, Amersham Buchler, FRG) by nick translation (nick translation kit, Amersham Buchler, FRG) was hybridized to Northern filters at a concentration of 5 ng per ml of hybridization mixture (5 x SSC; 1 x Denhardt's; 20 mM sodium phosphate pH 6.8; 0.1% SDS and 100 µg yeast tRNA [Boehringer-Mannheim, FRG] per ml) at 68 C for 12 to 14 hours. Membranes were then washed as described (Keil et al., 1984) and exposed at -70 °C to Kodak X-Omat AR films for varying times using Agfa Curix MR 800 intensifying screens.

The 0.8 kb nucleic acid sequence hybridized not only to intact HCV RNA but also to degradation products thereof. The 0.8 kb nucleic acid sequence did not hybridize to the 1.8 kb nucleic acid sequence, indicating that these two nucleic acid sequences correspond with fragments of the HCV genome which are not located in the same region of the genomic RNA.

Nucleotide sequencing. Subcloning of HCV specific phage DNA inserts into plasmid pEMBL 18 plus was done according to standard procedures (Maniatis et al., 1982). Single-stranded DNA of recombinant pEMBL plasmids was prepared as described (Dente et al., 1985). Dideoxy sequencing reactions (Sanger et al., 1977) were carried out as recommended by the supplier (Pharmacia, Sweden).

### Example 2

### Molecular cloning and nucleotide sequence of the genome of HCV

RNA preparation, cDNA synthesis and cloning. RNA preparation, cDNA synthesis, size selection and ligation of co-precipitated cDNA and λgt10 DNA (1 µg EcoRI digested dephosphorylated arms, Promega, USA) were done as described above. In vitro packaging of phage DNA using Packagene (Promega, USA) and titration of phages on E.coli strain C 600 HFL were performed as suggested by the supplier. The library was amplified once (Davis et al., 1986), and replicas transferred to nictrocellulose membranes (Amersham Buchler, FRG) (Benton and Davis, 1977) were hybridized with oligonucleotides as described above for Northern hybridization. Screening with cDNA fragments labeled with [α³²P] dCTP by nick translation (nick translation kit, Amersham Buchler, FRG) was done as described by Benton and Davis (1977). Positive clones were plaque purified and inserts subcloned into pEMBL plasmids (Maniatis et al., 1982; Dente et al., 1985; Davis et al., 1986).

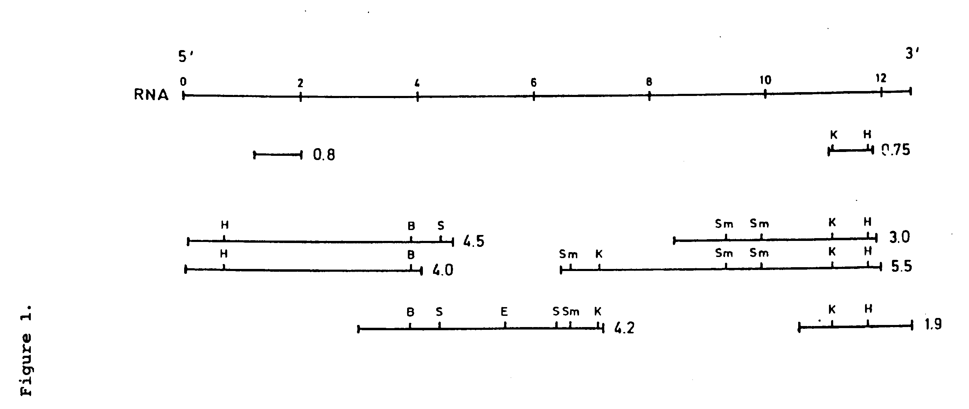

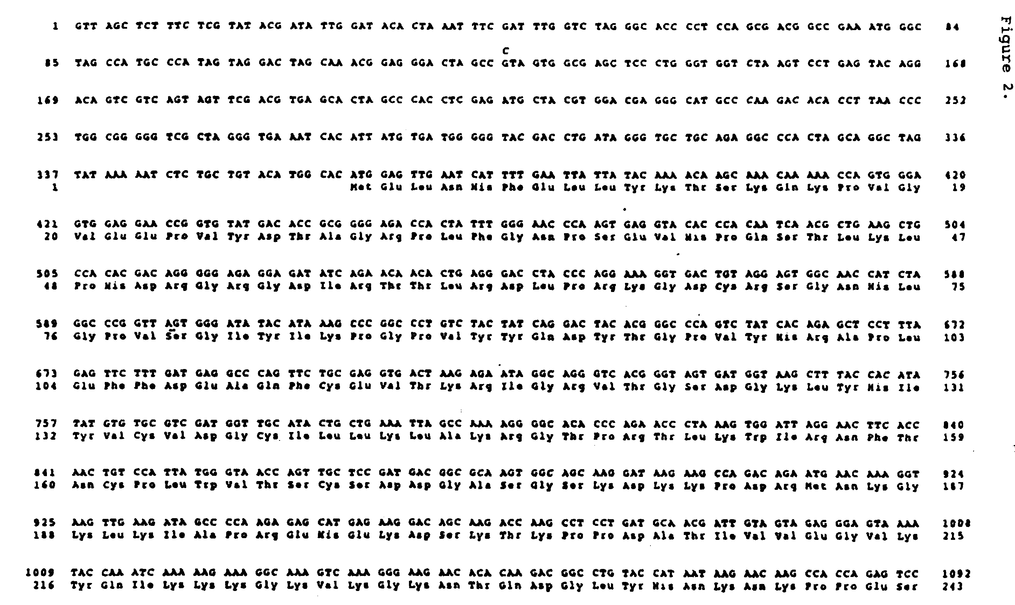

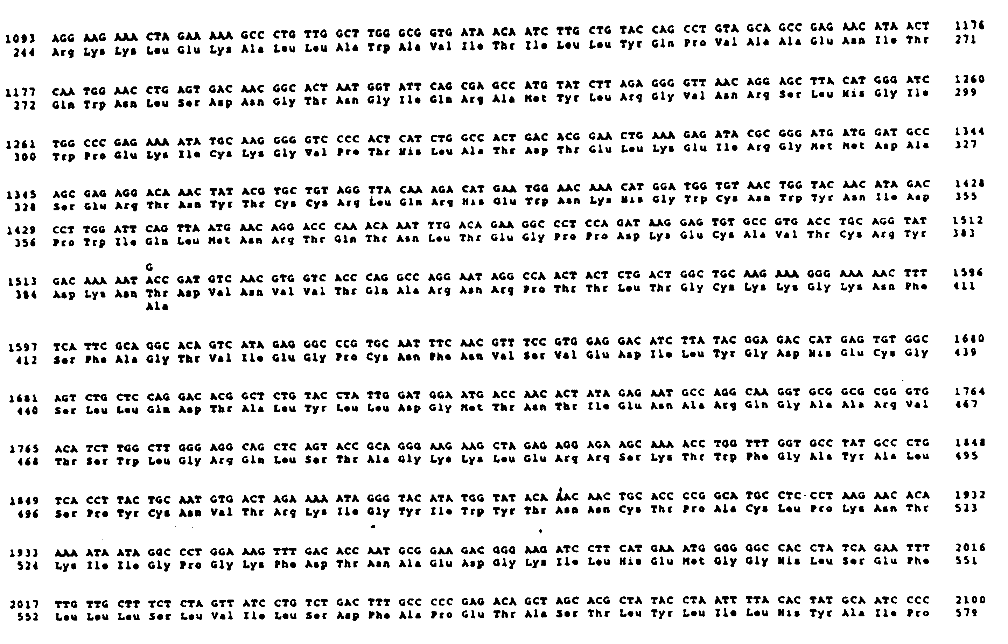

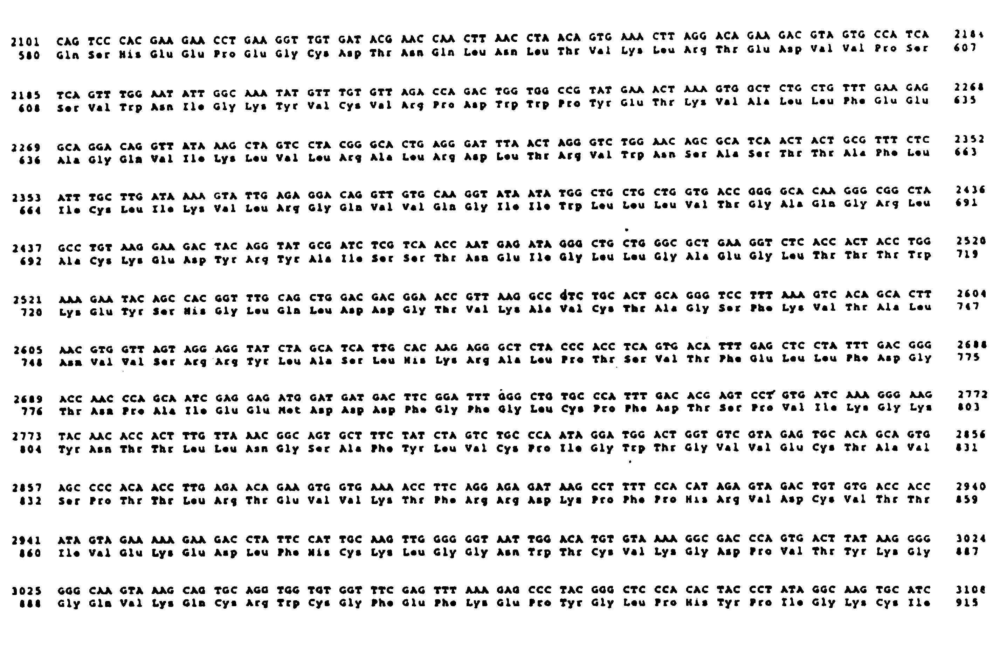

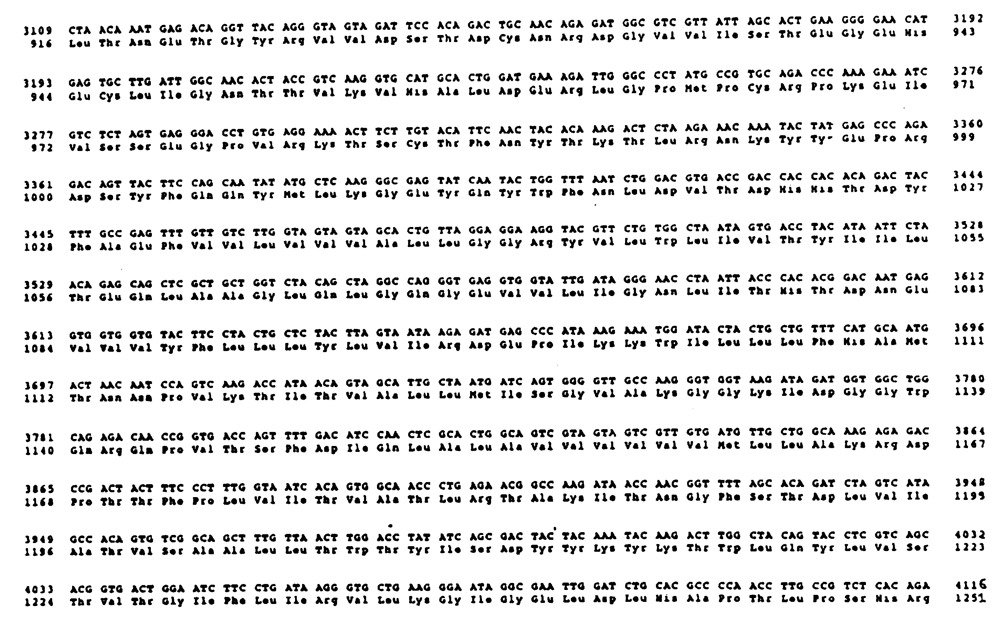

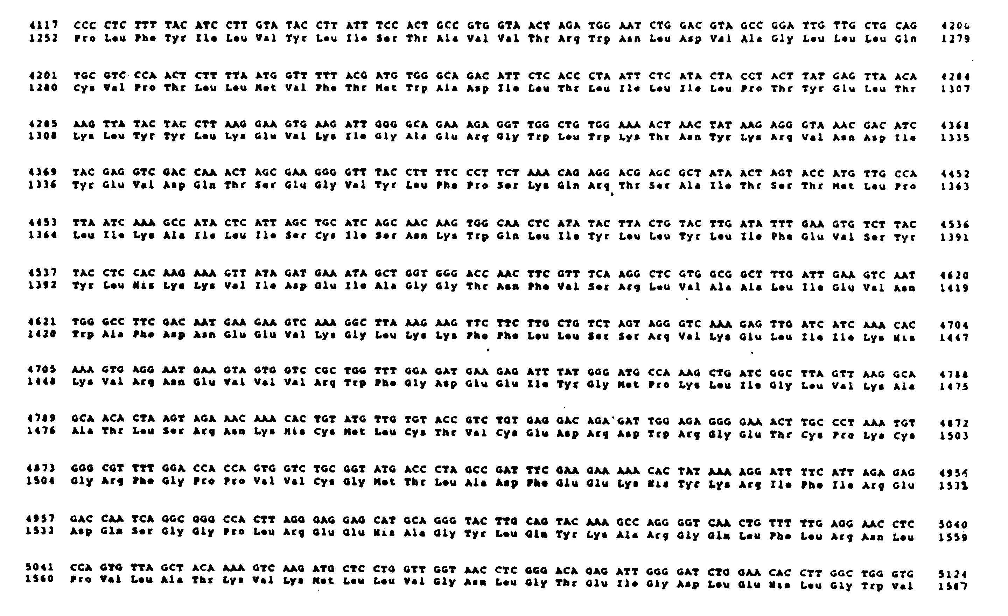

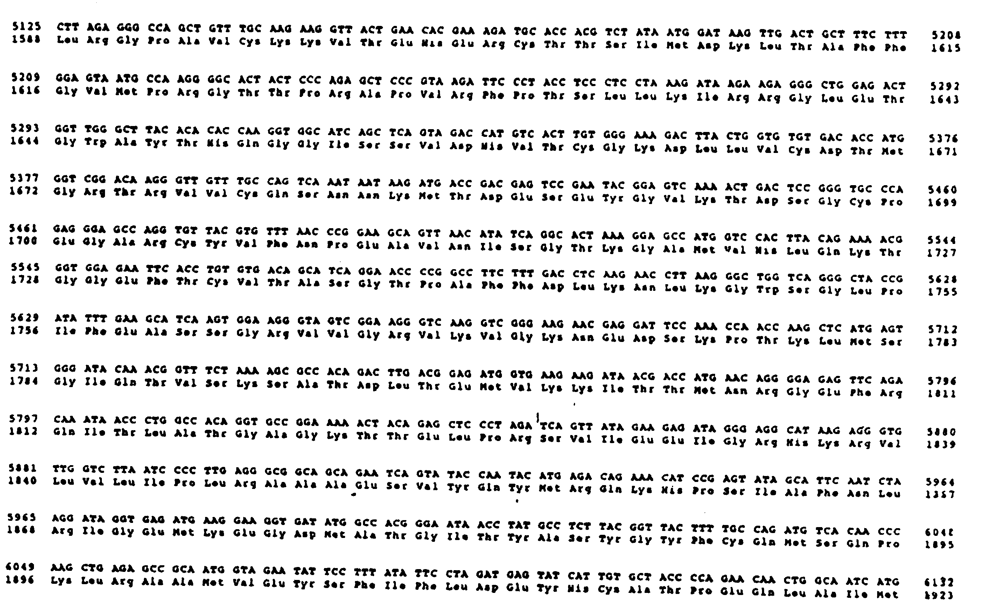

A ³²P 5'-end labeled oligonucleotide of 17 bases complementary to the RNA sequence encoding the amino acid sequence Cys Gly Asp Asp Gly Phe was used for screening a λgt10 cDNA library. This oligonucleotide which hybridized to the about 12 kb genomic RNA of HCV, identified inter alia a clone with an insert of 0.75 kb, which hybridized also to HCV RNA. This 0.75 kb nucleic acid sequence which represents a fragment of the HCV genome together with the 0.8 kb λgt11 nucleic acid sequence insert were used for further library screening resulting in a set of overlapping HCV nucleic acid sequences of which the relative positions and restriction site maps are shown in figure 1. These nucleic acid sequence fragments of the HCV genome are located between the following nucleic acid positions

| | |
|---|---|
| 4.0 kb fragment | 27-4027 |
| 4.5 kb fragment | 54-4494 |
| 0.8 kb fragment | 1140-2002 |
| 4.2 kb fragment | 3246-7252 |
| 5.5 kb fragment | 6656-11819 |

and within about the following nucleic acid positions

| | |
|---|---|
| 3.0 kb fragment | 8920-11920 |
| 1.9 kb fragment | 10384-12284 |
| 0.75 kb fragment | 10913-11663 |

Nucleotide sequencing. For complete nucleotide sequence determination exonuclease III and nuclease S1 (enzymes from Boehringer Mannheim, FRG) were used to establish deletion libraries of HCV derived cDNA inserts subcloned into pEMBL 18+ or 19+ plasmids (Hennikoff, 1987). Dideoxy sequencing (Sanger et al. 1977) of single stranded (Dente et al., 1985) or double stranded DNA templates was carried out using the T7 polymerase sequencing kit (Pharmacia, Sweden).

From the cDNA fragments a continuous sequence of 12284 nucleotides in length could be determined as shown in figure 2. This sequence contains one long open reading frame (ORF), starting with the ATG codon at position 364 to 366 and ending with TGA as a translational stop codon at 12058 to 12060. This ORF consists of 3898 codons capable of encoding a 435 kDa protein with an amino acid sequence shown in figure 2. Three nucleotide exchanges were detected as a result of differences in nucleotide sequence caused by possible heterogenicity of the virus population, two of which resulted in changes in the deduced amino acid sequence (figure 2).

It is concluded that almost the complete HCV genome has been cloned and sequenced by the procedures described above.

The 0.8 kb λgt11 nucleic acid sequence encoding an immunogenic HCV polypeptide identified with anti HCV serum was partially sequenced (see figure 3) which revealed that this sequence is located within 1.2 and 2.0 kb on the HCV RNA.

### Example 3

### Molecular cloning and expression of fusion proteins of HCV

cDNA fragments derived from two regions of the HCV genome, i.e. the 0,8 kb λgt11 insert of example 1 encoding amino acids 262-546 (see figure 2) and the nucleic acid sequence encoding amino acids 747-1071 (figure 2), are expressed as fusion proteins in the pEx system (Strebel, K. et al., 1986).

Bacterial extracts were separated by SDS-PAGE and stained according to standard procedures, and then tested for reactivity with the goat anti-HCV serum of example 1 in a Western blot.

The HCV specific fusion proteins were partially purified by SDS-PAGE and transfered to nitrocellulose and incubated with the goat anti-HCV serum. Specific antibodies against said fusion proteins were obtained after elution.

Antibodies specific for the above-mentioned fusion proteins were employed in a radio-immuno precipitation assay.

### Results

Both fusion proteins expressed in the pEx system were clearly identified as HCV specific after reaction with the goat anti-HCV serum.

Monospecific antiserum prepared against both fusions proteins precipitated HCV glycoproteins.

Antibodies specific for the 262-546-fusion protein precipitated the 44/48 kD and 33 kD protein, antibodies specific for the 747-1071-fusion protein precipitated the 55 kD protein from virus infected cells.

### Example 4

### Molecular cloning and expression of structural proteins via vaccinia virus

A fragment of the 4,0 kb clone shown in figure 1 (pHCK11) is prepared starting at the Hinfl restriction site (nucleotide 372) and ending at an artificial EcoRI site (nucleotide 4000) (Maniatis et al. 1982). For the 5' end an oligonucleotide adaptor was synthesized which contained an overhang compatible to BamHI, the original ATG(364-366) as translational start codon and a protruding end compatible to Hinfl at the 3' end. 
5' GATCCACCATGGAGTT HinfI
BamHI GTGGTACCTCAACTTA 5'
 At the 3' end of the construct a translational stop codon was introduced by deletion of the EcoRI protruding end with Mung bean nuclease and ligation into a blunt-end Stul/EcoRI adaptor residue: 
5' GCCTGAATTC 3'EcoRI
CGGACTTAAG
 (Maniatis et al. 1982).

Prior to inserting above-mentioned HCV sequences into vaccinia virus the heterologous gene is cloned into a recombination vector. For this purpose a pGS62 plasmid (Cranage, M.P. et al. 1986) was used which contains a cloning site downstream the P7.5K promotor within the 4.9kb thymidine kinase sequence. The cloning site comprises three unique restriction sites, BamHI, Smal and EcoRI. The recombination vector pGS62-3.8 was established by ligation of the described HCV sequence (372-4000) together with the adaptors into the BamHI/EcoRI digested pGS62.

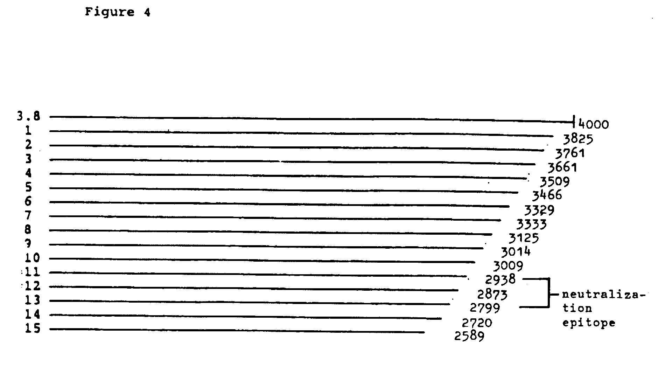

Based on the plasmid a set of 15 deletion mutants was established. By treatment with ExonucleaseIII (Hennikof et al., 1987) subsequent shortening of the HCV cDNA from the 3' end was performed. All deletions are located within the region coding for the HCV 55 kD protein by removal of about 100bp; most of the 55 kD protein is lost in mutant 15 ending at nucleotide 2589. ExoIII shortened cDNA clones were ligated into the pGS62 giving rise to pGS62-3.8Exo 1-15 (figure 4).

CVI cells were infected with vaccinia (strain Copenhagen, mutant TS7) at a MOI of 0.1. Three hours after infection pGS62-3.8 DNA as well as vaccinia WR DNA were transfected by the Ca3(PO<)2 precipitation method and incubated for two days. Virus progeny was harvested and selected for tk-phenotype on 143 tk-cells in the presence of brom-deoxy-Uridine (100 µg/ml). This selection was performed at least twice followed by two further cycles of plaque purification.

### Characterization of vaccinia-HCV recombinants

CVI cells were infected at an MOI between 2 and 10 with vaccinia-HCV recombinants and incubated for 8-16 hours. After fixation of the cells indirect immunofluorescence was performed using either monoclonal antibodies specific for HCV 55 kD protein or polyvalent anti-HCV sera. In all cases a cytoplasmatic fluorescence could be demonstrated.

After radioimmunoprecipitation and western blot analysis of cells infected with vaccinia recombinants four HCV-specific proteins were detected. By labeling with [³H] glucosamine it was shown that three of these proteins are glycosylated. The apparent molecular weights of these proteins were identical to those found in HCV infected cells with HCV specific sera, namely 20 kD(core), 44/48 kD, 33 kD and 55 kD.

Proteolytic processing and modifications appear to be authentic since HCV proteins produced by expression via vaccinia virus have the same apparent molecular weights as in HCV infected cells.

### Induction of neutralizing antibodies against HCV in mice.

Four groups of mice (3 mice/group) were infected once with

| | | | |
|---|---|---|---|
| a. | Vaccinia WR wildtype | (5x10⁶ pfu/individual) | **WR** |
| b. | Vaccinia 3.8 recombinant | (5x10⁷ pfu/individual) | **VAC3.8** |
| c. | Vaccinia 3.8Exo 4 (55 kD deleted) | (5x10⁷ pfu/individual) | **VAC3.8Exo 4** |
| d. | Vaccinia 3.8Exo 5 | (5x10⁷ pfu/individual) | **VAC3.8Exo 5** |
| e. | Vaccinia 3.8Exo 15 (55 kD deleted) | (5x10⁷ pfu/individual) | **VAC3.8Exo 15** |

by injection of purified virus intraperitoneally.
Mice were bled three weeks later. The reactivity of the sera was checked in a virus neutralization assay with HCV (Alfort) on PK[15] cells after serial dilution. (Rümenapf, T. et al. 1989).

| Neutralization titers | | |
|---|---|---|
| a. | WR | <1:2 |
| b. | VAC3.8 | 1:96 |
| | c. VAC3.8Exo 4 | 1:96 |
| d. | VAC3.8Exo 5 | <1:2 |
| e. | VAC3.8Exo 15 | <1.2 |

From the above it can be concluded that vaccinia virus containing a nucleic acid sequence comprising the genetic information for all structural proteins (VAC3.8) is able to induce virus neutralizing antibodies in mice, while incomplete constructs VAC3.8Exo 5-15 and WR are not.

As all deletions are located within the region coding for HCV 55 kD protein (most of the 55 kD protein is lost in mutant 15 ending at nucleotide 2589) and the other structural proteins are still being expressed by the recombinant vaccinia virus, it is clear that the 55 kD protein is responsible for the induction of HCV neutralizing antibodies.

### Example 5

### Immunization of pigs with VAC3.8

Out of three piglets (about 20 kg in weight) one animal (no. 28) was infected with wild type vaccinia virus (WR strain) and the other two (no. 26, 27) with recombinant VAC3.8 (i.p., i.v. and i.d., respectively). For infection 1x10⁸ pfu of vaccinia virus is applied to each animal.

Clinical signs in the course of vaccinia infection were apparent as erythema at the side of scarification and fever (41 °C) at day six after infection.
Titers against vaccinia and hog cholera virus:

Three weeks after infection the reactivity of the respective sera against vaccinia (WR on CVI cells) and HCV (Alfort on PK15 cells) was checked.

Neutralization was assayed after serial dilution of the sera by checking for complete absence of cpe (vaccinia) or specific signals in immunofluorescence (HCV). (Rümenapf, T. et al. 1989).

| Neutralization titers against vaccinia: | |
|---|---|
| pig 28 (WR) | 1:8 |
| pig 26 (VAC3.8) | 1:16 |
| pig 27 (VAC3.8) | 1:16 |

| Neutralization titers against HCV: | |
|---|---|
| pig 28 (WR) | <1:2 |
| pig 26 (VAC3.8) | 1:32 |
| pig 27 (VAC3.8) | 1:16 |

### Challenge with HCV:

Four weeks after immunization with vaccinia each of the pigs was challenged by infection with 5x10⁷ TCID₅₀ HCV Alfort. Virus was applicated oronasal according to the natural route of infection. This amount of virus has been experimentally determined to be compulsory lethal for pigs.

On day five after the challenge infection pig 28 revealed fever of 41.5 °C and kept this temperature until day 12. The moribund animal was killed that day expressing typical clinical signs of acute hog cholera.

Both pigs (26, 27) immunized with VAC3.8 did not show any sign of illness after the challenge with HCV for more than 14 days.

### Example 6

### Construction of a 55 kD protein expression vector

Clone pHCK11 is digested with restriction enzymes Sacl and Hpal according to standard techniques. The resulting 1.3 kb fragment, located between nucleotides 2672 (AGCTC) and 3971 (GTT) comprising most of HCV 55 kD protein, is isolated and cloned into the pseudorabies virus (PRV) gX gene (Maniatis et al. 1982).

Briefly, the cloned gX sequence was digested with Sacl and Apal. The Apal 5' protruding ends were made blunt by filling up with Klenow fragment. After ligation the putative gX leader peptide coding sequence was located just upstream of the inserted HCV 55 kD sequence.

A translational stop codon downstream the HCV sequence was introduced by digestion with Bgl II (Bgl II site: 3936-3941) and religation after filling up the overhangs with Klenow fragment. This construct was placed downstream of the PRV gX promotor (clone 16/4-1.3). Clone 16/4-1.3 was transfected into MDBK cells by the DEAE dextran method (Maniatis et al. 1989). 16 h. later cells were infected with PRV (m.o.i. = 1). 4 h. post infection cells were fixed with a mixture of cold (-20 C) methanol/acetone. Indirect immunofluorescence with monoclonal antibodies (MABs) anti-HCV 55 kD protein revealed a specific signal in 5-10% of the cells. PRV infected cells without transfection and cells only transfected with clone 16/4-1.3 did not show any signal in this assay.

### Brief description of the drawings

Fig. 1 displays physical maps of different HCV derived cDNA clones and their position relative to the RNA genome (upper line). Two HCV derived cDNA clones isolated after screening with either the antibody probe (0.8 kb clone) or the degenerated oligonucleotide probe (0.75 kb clone) are shown in the second line. The cDNA fragments chosen for nucleotide sequencing are indicated below. All numbers represent sizes of DNA fragments in kb. Restriction sites: B = Bgl II; E = EcoRI; H = Hind III; K = Kpn I; S = Sal I; Sm = Sma I.

Fig. 2 depicts a nucleic acid sequence of HCV and deduced amino acid sequence of the long open reading frame. Nucleotide exchanges between different cDNA clones and resulting changes in amino acid sequence are indicated. The part of the sequence corresponding to the oligonucleotide used for screening is underlined.

Fig. 3 shows the cDNA sequence from part of the 0.8 kb HCV insert of a λgt11 clone and the deduced amino acid sequence in one-letter code.

Fig. 4 shows the length of the HCV DNA cloned in the pGS62 vector. A set of 15 deletion mutants derived from cDNA clone pHCK11 was established by treatment with Exonuclease III and cloned in the pGS62 vector giving rise to pGS62-3.8Exo 1-15. 3' end nucleotides are indicated.

### References

BENTON, W., and DAVIS, R. (1977). Screening λgt recombinant clones by hybridization to single plaques in sity. Science 196, 180-182.
CHIRGWIN, J.M., PRZYBYLA, A.E., MAcDONALD, R.J., and RUTTER, W.J. (1979). Isolation of biologically active ribonucleic acid from sources enriched in ribonuclease, Biochemistry 18, 5294-5299.
CRANAGE, M.P. et al. (1986). EMBO, J. 5, 3057-3063.
DAVIS, L.G., DIBNER, M.D., and BATTEY, J.F. (1986). Basic Methods in Molecular Biology, 190-191.
Elsevier, New York, Amsterdam, London.
DENTE, L., SOLLAZZO, M., BALDARI, C., CESARENI, G., and CORTESE, R. (1985). The pEMBL family of single-stranded vectors. In: DNA Cloning, Vol. 1, (Glover, D.M., ed.), IRL Press Oxford/Washington DC, pp. 101-107.
HENNIKOFF, S. (1987). Unidirectional digestion with exonuclease III in DNA sequence analysis. In: Meth. Enzymol. (Wu, R., ed.) 155, 156-165.
HUYNH, T.V., YOUNG, R.A., and DAVIS, R.W. (1985). Constructing and screening cDNA libraries in λgt10 and λgt11. In: DNA Cloning: A Practical Approach, Vol. 2, (Glover, D.M., ed.), IRL Press Oxford, pp. 49-78.
KEIL, G.M., EBELING-KEIL, A., and KOSZINOWSKI, U.H. (1984). Temporal regulation of murine cytomegalovirus transcription and mapping of viral RNA synthesized at immediate early times after infection, J. Virol. 50, 784-795.
MANIATIS, T., FRITSCH, E.F., and SAMBROOKS, S. (1982). Molecular Cloning, a Laboratory Manual. Cold Spring Harbor Laboratory Press, Cold Spring Harbor, NY.
SANGER, F., NICKLEN, S., and COULSON, A.R. (1977). DNA sequencing with chain-terminating inhibitors. Proc. Natl. Acad. Sci. U.S.A. 74, 5363-5467.
MANIATIS, T. et al. (1989). Molecular Cloning, a Laboratory Manual, second edition, Cold Spring Harbor Laboratory Press, Cold Spring Harbor, NY.
STREBEL, K. et al. (1986). J. Virology 57, 983-991.
RüMENOPF, T. et al. (1989). Virology 171, 18-27.
YOUNG, R.A., and DAVIS, R.W. (1983). Efficient isolation of genes by using antibody probes. Proc. Natl. Acad. Sci. U.S.A. 80, 1194-1198.

## Claims (Claims for the following Contracting State(s): BE, CH, DK, FR, GB, IT, LI, NL, SE)

1. A nucleic acid sequence corresponding to a fragment of the hog cholera virus genome, and encoding a hog cholera virus polypeptide or an antigenic fragment thereof, capable of inducing virus neutralising antibodies, which polypeptide comprises the amino acid sequence located within the amino acid position 689-1067 shown in Fig 2 or is a functional equivalent thereof.

2. A nucleic acid sequence according to claim 1 characterised in that it comprises either the nucleotide sequence 2428-3564 shown in Fig 2 or a fragment thereof, or a nucleotide sequence which hybridises under stringent conditions to said nucleotide sequence shown in Fig 2.

3. A recombinant nucleic acid molecule comprising a vector nucleic acid molecule and a nucleic acid sequence according to claim 1 or claim 2.

4. A host cell capable of producing a polypeptide of hog cholera virus comprising a recombinant nucleic acid molecule according to claim 3.

5. A host cell according to claim 4 characterised in that the host cell is a bacterium.

6. A recombinant virus containing a recombinant nucleic acid molecule according to claim 3.

7. A method for producing a hog cholera virus polypeptide which polypeptide corresponds to a portion of the hog cholera virus precursor polyprotein, capable of inducing virus neutralising antibodies, wherein a host cell according to claim 4 or 5, or a recombinant virus according to claim 6 is propagated in a culture under conditions whereby the polypeptide is expressed, whereafter the polypeptide is isolated from the culture.

8. A vaccine for the protection of animals against hog cholera virus infection, characterised in that it comprises a hog cholera virus polypeptide which polypeptide corresponds to a portion of the hog cholera virus precursor polyprotein, capable of inducing virus neutralising antibodies, which polypeptide comprises the amino acid sequence located within the amino acid position 689-1067 shown in Fig 2 *in non-denatured and non-reduced form,* or is an antigenic fragment or functional equivalent thereof.

9. A vaccine for the protection of animals against hog cholera virus infection, characterised in that it comprises a hog cholera virus polypeptide *as obtainable by expression in a recombinant host cell of* a nucleic acid sequence corresponding to a fragment of the hog cholera virus genome, and encoding a hog cholera virus polypeptide or an antigenic fragment thereof, capable of inducing virus neutralising antibodies, which polypeptide comprises the amino acid sequence located within the amino acid position 689-1067 shown in Fig 2 or is a functional equivalent thereof.

10. A vaccine according to claim 9, wherein said nucleic acid sequence comprises either the nucleotide sequence 2428-3564 shown in Fig 2 or a fragment thereof, or a nucleotide sequence which hybridises under stringent conditions to said nucleotide sequence shown in Fig 2.

11. A vaccine for the protection of animals against hog cholera virus infection, characterised in that it comprises a host cell according to claims 4-5 or a recombinant virus according to claim 6.

12. A method for the preparation of a hog cholera virus vaccine, characterised in that a polypeptide as defined in claims 8-10 is formed to a pharmaceutical preparation with immunising activity.

13. A method for the preparation of a hog cholera vaccine, characterised in that a host cell according to claims 4-5 or a recombinant virus according to claim 6 is propagated in a culture, whereafter the host cell or the recombinant virus is harvested and is formed to a pharmaceutical preparation with immunising activity.

## Claims (Claims for the following Contracting State(s): ES, GR)

1. A process for the preparation of a recombinant nucleic acid molecule which comprises incorporating a nucleic acid sequence corresponding to a fragment of the hog cholera virus genome, and encoding a hog cholera virus polypeptide or an antigenic fragment thereof, capable of inducing virus neutralizing antibodies, which polypeptide comprises the amino acid sequence located within the amino acid position 689-1067 shown in figure 2 or is a functional equivalent thereof, in a vector nucleic acid molecule.

2. A process according to claim 1, characterized in that said nucleic acid sequence comprises, either the nucleotide sequence 2428-3564 shown in figure 2 or a fragment thereof, or a nucleotide sequence which hybridizes under stringent conditions to said nucleotide sequence shown in figure 2.

3. A process which comprises expressing in a host cell the nucleic acid sequence defined in claim 1 or 2.

4. A process according to claims 3, characterized in that the host cell is a bacterium.

5. A process for the preparation of a recombinant virus, characterized in that the nucleic acid sequence defined in claim 1 or 2 is incorporated into a virus.

6. A process according to claims 3 or 4 which comprises allowing expression of the nucleic acid sequence in a culture, and recovering the polypeptide from the culture.

7. A process for the preparation of a vaccine for the protection of animals against hog cholera virus infection comprising, following the process of claim 6, the forming of the polypeptide expression product to a pharmaceutical preparation with immunizing activity.

8. A process for the preparation of a vaccine for the protection of animals against hog cholera virus infection comprising forming a host cell capable of expressing the nucleic acid sequence defined in claims 1 or 2, or a recombinant virus comprising the nucleic acid sequence defined in claims 1 or 2, to a pharmaceutical preparation with immunizing activity.

## Patentansprüche (Patentansprüche für folgende(n) Vertragsstaat(en): BE, CH, DE, DK, FR, GB, IT, LI, NL, SE)

1. Nukleinsäuresequenz, die einem Fragment des Schweinepestvirusgenoms entspricht und ein Schweinepestvirus-Polypeptid oder antigenisches Fragment desselben codiert, das dazu fähig ist, virusneutralisierende Antikörper zu induzieren, wobei das Polypeptid die in der in Figur 2 gezeigten Aminosäureposition 689-1067 vorliegende Aminosäuresequenz umfaßt oder ein funktionelles Äquivalent davon ist.

2. Nukleinsäuresequenz gemäß Anspruch 1, dadurch gekennzeichnet, daß sie entweder die in Figur 2 dargestellte Nukleotidsequenz 2428-3564 oder ein Fragment derselben oder eine Nukleotidsequenz, die unter strengen Bedingungen mit der in Figur 2 dargestellten Nukleotid-sequenz hybridisiert, enthält.

3. Rekombinantes Nukleinsäuremolekül mit einem Vektornukleinsäuremolekül und einer Nukleinsäuresequenz gemäß Anspruch 1 oder 2.

4. Wirtszelle, die dazu fähig ist, ein Schweinepestvirus-Polypeptid mit einem rekombinanten Nukleinsäuremolekül gemäß Anspruch 3 zu produzieren.

5. Wirtszelle gemäß Anspruch 4, dadurch gekennzeichnet, daß es sich dabei um ein Bakterium handelt.

6. Rekombinantes Virus mit einem rekombinanten Nukleinsäuremolekül gemäß Anspruch 3.

7. Verfahren zur Herstellung eines Schweinepestvirus-Polypeptids, das einem Teil des Schweinepestvirus-Vorläuferpolyproteins entspricht und dazu fähig ist, virusneutralisierende Antikörper zu induzieren, bei dem eine Wirtszelle gemäß Anspruch 4 oder 5 oder ein rekombinantes Virus gemäß Anspruch 6 in einer Kultur unter Bedingungen, unter denen das Polypeptid expremiert wird, gezüchtet wird, wonach man das Polypeptid aus der Kultur isoliert.

8. Impfstoff zum Schutz von Tieren gegen Infektion mit Schweinepestvirus, dadurch gekennzeichnet, daß er ein Schweinepestvirus-Polypeptid enthält, das einem Teil des Schweinepestvirus-Vorläuferpolyproteins entspricht und dazu fähig ist, virusneutralisierende Antikörper zu induzieren, wobei das Polypeptid die innerhalb der in Figur 2 dargestellten Aminosäureposition 689-1067 befindliche Aminosäuresequenz *in nichtdenaturierter und nichtreduzierter Form* enthält oder ein antigenisches Fragment oder funktionelles Äquivalent davon ist.

9. Impfstoff zum Schutz von Tieren gegen Infektion mit Schweinepestvirus, dadurch gekennzeichnet, daß er ein Schweinepestvirus-Polypeptid in der Form enthält, *wie es durch Expression* in *einer rekombinanten Wirtszelle einer* Nukleinsäuresequenz *erhalten werden kann,* die einem Fragment des Schweinepestvirus-Genoms entspricht und ein Schweinepestvirus-Polypeptid oder antigenes Fragment davon codiert, das fähig ist, virusneutralisierende Antikörper zu induzieren, wobei das Polypeptid die innerhalb der in Figur 2 dargestellten Aminosäureposition 689-1067 befindliche Aminosäuresequenz umfaßt oder ein funktionelles Äquivalent davon ist.

10. Impfstoff gemäß Anspruch 9, wobei die Nukleinsäuresequenz entweder die in Figur 2 dargestellte Nukleotidsequenz 2428-3564 bzw. ein Fragment davon oder eine Nukleotidsequenz, die unter strengen Bedingungen mit der in Figur 2 dargestellten Nukleotidsequenz hybridisiert, enthält.

11. Impfstoff zum Schutz von Tieren gegen Infektion mit Schweinepestvirus, dadurch gekennzeichnet, daß er eine Wirtszelle gemäß Ansprüchen 4-5 oder ein rekombinantes Virus gemäß Anspruch 6 enthält.

12. Verfahren zur Herstellung eines Schweinepestvirus-Impfstoffs, dadurch gekennzeichnet, daß ein wie in den Ansprüchen 8-10 definiertes Polypeptid als pharmazeutische Zusammensetzung mit immunisierender Wirksamkeit formuliert wird.

13. Verfahren zur Herstellung eines Schweinepest-Impfstoffs, dadurch gekennzeichnet, daß eine Wirtszelle gemäß den Ansprüchen 4-5 oder ein rekombinantes Virus gemäß Anspruch 6 in einer Kultur gezüchtet wird, wonach die Wirtszelle bzw. das rekombinante Virus geerntet wird und als pharmazeutische Zusammensetzung mit immunisierender Wirksamkeit formuliert wird.

## Patentansprüche (Patentansprüche für folgende(n) Vertragsstaat(en): ES, GR)

1. Ein Verfahren zur Herstellung eines rekombinanten Nukleinsäuremoleküls, welches die Einverleibung einer Nukleinsäuresequenz entsprechend einem Fragment des Schweinepestvirusgenoms und das Codieren eines Schweinepestviruspolypeptids oder eines antigenen Fragmentes davon, das fähig ist, virusneutralisierende Antikörper zu induzieren, wobei das Polypeptid die innerhalb der n Figur 2 dargestellten Aminosäurestellung 689-1067 liegende Aminosäuresequenz umfasst oder ein funktionelles Aequivalent davon ist, in ein Vektornukleinsäuremolekül umfasst.

2. Ein Verfahren nach Anspruch 1, dadurch gekennzeichnet, dass die genannte Nukleinsäuresequenz entweder die in Figur 2 dargestellte Nukleotid-Sequenz 2428-3564 oder ein Fragment davon oder eine Nukleotid-Sequenz, welche unter stiringenten Bedingungen zu der genannten, in Figur 2 dargestellten Nukleotidsequenz hybridisiert, umfasst.

3. Ein Verfahren, welches die Expression der in Anspruch 1 oder 2 definierten Nukleinsäuresequenz in eine Wirtszelle umfasst.

4. Ein Verfahren nach Anspruch 3, dadurch gekennzeichnet, dass die Wirtszelle ein Bakterium ist.

5. Ein Verfahren zur Herstellung eines rekombinanten Virus, dadurch gekennzeichnet, dass die in Anspruch 1 oder 2 definierte Nukleinsäuresequenz in einen Virus einverleibt wird.

6. Ein Verfahren nach Anspruch 3 oder 4, welches das Exprimierenlassen der Nukleinsäuresequenz in eine Kultur und das Gewinnen des Polypeptids aus der Kultur umfasst.

7. Ein Verfahren zur Herstellung eines Impistoffes zum Schutz von Tieren gegen SchweinepestvirusInfektion, umfassend, im Anschluss an das Verfahren nach Anspruch 6, die Verarbeitung des Polypeptid-Expressionsproduktes zu einem pharmazeutischen Präparat mit immunisierender Wirkung.

8. Ein Verfahren zur Herstellung eines Impistoffes zum Schutz von Tieren gegen Schweinepestvirus-Infektion, umfassend die Verarbeitung einer Wirtszelle, die fähig ist, die in Anspruch 1 oder 2 definierte Nukleinsäuresequenz zu exprimieren, oder eines rekombinanten Virus, welcher die in Anspruch 1 oder 2 definierte Nukleinsäuresequenz umfasst, zu einem pharmazeutischen Präparat mit immunisierender Wirkung.

## Revendications (Revendications pour l'(les) Etat(s) contractant(s) suivant(s): BE, CH, DE, DK, FR, GB, IT, LI, NL, SE)

1. Séquence d'acide nucléique correspondant à un fragment du génome du virus de la peste porcine, et codant pour un polypeptide du virus de la peste porcine ou un fragment antigénique de celui-ci, susceptible d'induire des anticorps de neutralisation de virus, lequel polypeptide comprend la séquence d'acides aminés localisée au sein de la position des acides aminés 689-1067 représentée à la fig. 2 ou est un équivalent fonctionnel de celle-ci.

2. Séquence d'acide nucléique selon la revendication 1, caractérisée en ce qu'elle comprend soit la séquence nucléotidique 2428-3564 représentée à la fig. 2 ou un fragment de celle-ci, soit une séquence nucléotidique s'hybridant dans des conditions stringentes à ladite séquence nucléotidique représentée à la fig. 2.

3. Molécule d'acide nucléique recombinant comprenant une molécule d'acide nucléique vecteur et une séquence d'acide nucléique selon la revendication 1 ou la revendication 2.

4. Cellule hôte susceptible de produire un polypeptide du virus de la peste porcine comprenant une molécule d'acide nucléique recombinant selon la revendication 3.

5. Cellule hôte selon la revendication 4, caractérisée en ce que la cellule hôte est une bactérie.

6. Virus recombinant contenant une molécule d'acide nucléique recombinant selon la revendication 3.

7. Procédé de production d'un polypeptide du virus de la peste porcine, lequel polypeptide correspond à une portion de la polyprotéine du précurseur du virus de la peste porcine, susceptible d'induire des anticorps de neutralisation de virus, caractérisé en ce qu'une cellule hôte selon la revendication 4 ou 5, ou un virus recombinant selon la revendication 6 est propagé en culture dans des conditions telles que le polypeptide est exprimé, puis le polypeptide est isolé de la culture.

8. Vaccin destiné à protéger les animaux contre l'infection par le virus de la peste porcine, caractérisé en ce qu'il comprend un polypeptide du virus de la peste porcine, lequel polypeptide correspond à une portion de la polyprotéine précurseur du virus de la peste porcine, susceptible d'induire des anticorps de neutralisation de virus, lequel polypeptide comprend la séquence d'acides aminés localisée au sein de la position des acides aminés 689-1067 représentée à la fig. 2 *sous forme non dénaturée et non réduite*, ou est un fragment antigénique ou un équivalent fonctionnel de celle-ci.

9. Vaccin destiné à protéger les animaux contre l'infection par le virus de la peste porcine, caractérisé en ce qu'il comprend un polypeptide du virus de la peste porcine tel qu'on peut l'obtenir par l'expression dans une cellule hôte recombinante d'une séquence d'acide nucléique correspondant à un fragment du génome du virus de la peste porcine, et codant pour un polypeptide du virus de la peste porcine ou un fragment antigénique de celui-ci, susceptible d'induire des anticorps de neutralisation de virus, lequel polypeptide comprend la séquence d'acides aminés localisée au sein de la position des acides aminés 689-1067 représentée à la fig. 2 ou est un équivalent fonctionnel de celle-ci.

10. Vaccin selon la revendication 9, caractérisé en ce que ladite séquence d'acide nucléique comprend soit la séquence nucléotidique 2428-3564 représentée à la fig. 2 ou un fragment de celle-ci, soit une séquence nucléotidique s'hybridant dans des conditions stringentes à ladite séquence nucléotidique représentée à la fig. 2.

11. Vaccin destiné à protéger les animaux contre l'infection par le virus de la peste porcine, caractérisé en ce qu'il comprend une cellule hôte selon les revendications 4-5 ou un virus recombinant selon la revendication 6.

12. Procédé de préparation d'un vaccin du virus de la peste porcine, caractérisé en ce qu'un polypeptide tel que défini dans les revendications 8-10 est formé en une préparation pharmaceutique à activité immunisante.

13. Procédé de préparation d'un vaccin du virus de la peste porcine, caractérisé en ce que la cellule hôte selon les revendications 4-5 ou un virus recombinant selon la revendication 6 est propagé en culture, puis la cellule hôte ou le virus recombinant est récolté et formé en une préparation pharmaceutique à activité immunisante.

## Revendications (Revendications pour l'(les) Etat(s) contractant(s) suivant(s): ES, GR)

1. Un procédé de préparation d'une molécule d'acide nucléique recombinante qui comprend l'incorporation d'une séquence nucléotidique correspondant à un fragment du génome du virus de la peste porcine, et codant pour un polypeptide du virus de la peste porcine ou un fragment antigénique de ce dernier, capable d'induire des anticorps neutralisants le virus, lequel polypeptide comprend la séquence d'acides aminés localisée entre les positions d'acide aminé 689-1067 décrite à la figure 2 ou correspond à un équivalent fonctionnel de ce dernier, dans une molécule d'acide nucléique vecteur.

2. Un procédé selon la revendication 1 caractérisé en ce que ladite séquence nucléotidique comprend soit la séquence nucléotidique 2428-3564 décrite à la figure 2 soit un fragment de cette dernière soit une séquence nucléotidique qui s'hybride dans des conditions drastiques à ladite séquence nucléotidique décrite à la figure 2.

3. Un procédé qui comprend l'expression dans une cellule hôte de la séquence d'acides nucléiques définie dans la revendication 1 ou 2.

4. Un procédé selon la revendication 3 caractérisée en ce que la cellule hôte est une bactérie.

5. Un procédé de préparation d'un virus recombinant caractérisé en ce que la séquence d'acides nucléiques définie dans la revendication 1 ou 2 est incorporée dans un virus.

6. Un procédé selon la revendication 3 ou 4 qui comprend l'expression de la séquence d'acides nucléiques dans une culture et la récupération du polypeptide à partir de la culture.

7. Un procédé de préparation d'un vaccin pour la protection des animaux contre une infection par le virus de la peste porcine comprenant. en suivant le procédé de la revendication 6, la transformation du produit d'expression du polypeptide en une préparation pharmaceutique présentant une activité immunisante.

8. Un procédé de préparation d'un vaccin pour la protection des animaux contre une infection par le virus de la peste porcine comprenant la transformation d'une cellule hôte capable d'exprimer la séquence d'acides nucléiques définie dans les revendications 1 ou 2 ou d'un virus recombinant comprenant la séquence d'acides nucléiques définie dans les revendications 1 ou 2, en une composition pharmaceutique présentant une activité immunisante.
